# EUROPEAN PATENT APPLICATION

(11) **EP 1 466 984 A1**
(43) Date of publication of application: **13.10.2004**
(21) Application number: 02715803.9
(22) Date of filing: 18.01.2002
(51) Int. Cl.: C12P 7/42, C12P 13/00, C12N 1/20

(54) **PROCESS FOR PRODUCING A-HYDROXY ACID AMMONIUM SALT**

(71) Applicant: NIPPON SODA CO., LTD., Chiyoda-ku, Tokyo 100-8165 (JP)
(72) Inventor: KOBAYASHI, Y., c/o NipponSoda Co., Ltd., Odawara-shi, Kanagawa 250-0280 (JP); MAEDA, Kasumi, c/o Nippon Soda Co., Ltd., Odawara-shi, Kanagawa 250-0280 (JP)
(74) Representative: Williams, Aylsa
(86) International application number: PCT/JP2002/000312
(87) International publication number: WO 2003/062437

(57) **Abstract**

The present invention provides a method for producing a-hydroxy acid ammonium salt that uses a specific microbial strain capable of accumulating α-hydroxy acid ammonium salt at a high concentration while also maintaining an industrially satisfactory production rate for a long period of time. More specifically, the present invention uses a microbial catalyst originating in a microbial strain which, during conversion of α-hydroxynitrile to α-hydroxy acid ammonium salt, is capable of maintaining the average production rate of α-hydroxy acid ammonium salt at at least 100 µmol/min per g of dry microbial cell weight for 14 days or more without adding fresh microbial catalyst, and is capable of accumulating the α-hydroxy acid ammonium salt at 20 to 60% by weight. An example of this microbial strain is Arthrobacter sp. strain NSSC204.

## Description

### Technical Field

The present invention relates to a process for producing α-hydroxy acid ammonium salt by hydrolyzing α-hydroxynitrile using a microbial catalyst originating in a specific microbial strain, and a microbial strain used in said process. α-hydroxy acid ammonium salt can be derived to free α-hydroxy acid by ordinary methods. Among α-hydroxy acids and α-hydroxy acid ammonium salts, lactic acid and ammonium lactate are used in the food industry, brewing industry, pharmaceutical industry and the like, and 2-hydroxy-4-methylthiobutyric acid and ammonium 2-hydroxy-4- methylthiobutyrate are useful as livestock feed additives, while α-hydroxyisobutyric acid is useful as a raw material for organic syntheses.

### Background Art

Known examples of methods for producing α-hydroxy acid from α-hydroxynitrile using microbes include a production method of lactic acid, glycolic acid and the like using microbes belonging to the genera Bacillus, Bacteridium, Micrococcus and Brevibacterium (Japanese Examined Patent Application, Second Publication No. 58-15120), a production method of lactic acid, glycolic acid and 2-hydroxyisobutyric acid using microbes belonging to the genus Corynebacterium (Japanese Unexamined Patent Application, First Publication No. 61-56086), a production method of lactic acid, 2-hydroxyisobutyric acid, 2-hydroxy-2-hydroxyphenylpropionic acid and mandelic acid using microbes belonging to the genera Pseudomonas, Arthrobacter, Aspergillus, Penicillium, Cochliobolus and Fusarium (Japanese Unexamined Patent Application, First Publication No. 63-222696), a production method of 2-hydroxy-3,3-dimethyl-4-butyrolactone using microbes belonging to the genera Arthrobacter, Aspergillus, Bacillus, Bacteridium, Brevibacterium, Cochlioborus, Corynebacterium, Micrococcus, Nocardia, Penicillium, Pseudomonas and Fusarium (Japanese Unexamined Patent Application, First Publication No. 64-10996), a production method of 2-hydroxyisobutyric acid using microbes belonging to the genera Rhodococcus, Pseudomonas, Arthrobacter and Brevibacterium (Japanese Unexamined Patent Application, First Publication No. 4-40897), a production method of 2-hydroxy-4-methylthiobutyric acid using microbes belonging to the genera Caseobacter, Pseudomonas, Alcaligenes, Corynebacterium, Brevibacterium, Nocardia, Rhodococcus and Arthrobacter (Japanese Unexamined Patent Application, First Publication No. 4-40898), a production method of α-hydroxy-4-methylthiobutyric acid using microbes belonging to the genera Pantoea, Micrococcus, Bacteridium, Bacillus and the like (Japanese Unexamined Patent Application, First Publication No. 8-173175), and a production method of α-hydroxy-4-methylthiobutyric acid using Alcaligenes faecalis ATCC 8750, Rhodococcus sp. HT29-7 or Gordona terrae MA-1 (WO96-09403). In the production method of α-hydroxy acid disclosed in these prior documents, an α-hydroxy acid ammonium salt is first obtained by hydrolyzing α-hydroxynitrile with a microbial catalyst, and then free α-hydroxy acid is produced from the salt in accordance with ordinary methods such as acid neutralization, ion exchange resin column, electrodialysis or thermal decomposition.

None of these α-hydroxy acid production method are satisfactory in terms of forming and accumulating the target substances at high concentrations, and the production rate of the target substances per microbial catalyst cannot be said to be satisfactory from an industrial standpoint. For example, it is described in the aforementioned Japanese Unexamined Patent Application, First Publication No. 61-56086 that the cumulative concentration of lactic acid using microbes belonging to the genus Corynebacterium is 9.8% by weight and the production rate is 257 mmol/min/g of dry microbial cell weight (30°C/4 hours), it is described in Japanese Unexamined Patent Application, First Publication No. 63-222696 that the cumulative concentration of lactic acid using microbes belonging to the genus Pseudomonas is 10% by weight, the cumulative concentration of lactic acid using microbes belonging to the genus Arthrobacter is 0.15% by weight and the cumulative concentration of 2-hydroxyisobutyric acid using microbes belonging to the genus Pseudomonas is 0.8% by weight, it is described in Japanese Unexamined Patent Application, First Publication No. 4-40898 that the cumulative concentration of 2-hydroxy-4-methylthiobutyric acid using Caseobacter sp. strain BC23 is 188 mM (2.8% by weight) and the production rate is 4.7 mmol/h/450.D.630 nm (25°C), it is described in Japanese Unexamined Patent Application, First Publication No. 8-173175 that the cumulative concentration of 2-hydroxy-4-methylthiobutyric acid using Bacteridium sp. R341 (FERM-P2719) is 0.79% by weight, and it is described in WO96-09403 that the cumulative concentration of 2-hydroxy-4-methylthiobutyric acid using Alcaligenes faecalis strain ATCC 8750 is 940 mmol/L (14.1% by weight) and the production rate is 8.7 mmol/min/g of dry microbial cell weight (25°C/180 hours), and that the cumulative concentration of 2-hydroxy-4-methylthiobutyric acid using Gordona terrae strain MA-1 is 1.5 mol/L (22.5% by weight) and the production rate is 250 mmol/min/g of dry microbial cell weight (35°C, initial rate).

Examples of reasons for the low cumulative concentrations of products as indicated above include the α-hydroxynitrile partially dissociating to hydrocyanic acid and the corresponding aldehyde or ketone in aqueous solution (Chemical Reviews, Vol. 42, p. 189, 1948), and enzyme activity being inhibited by hydrocyanic acid (Agricultural Biological Chemistry, Vol. 46, p. 165, 1982). In addition, the possibility has also been pointed out that enzyme is deactivated in a short period of time by the action of the dissociated aldehyde, and examples of methods that have been proposed for solving this problem include a method including the addition of acidic sulfite ion or dithionite ion (Japanese Unexamined Patent Application, First Publication No. 5-192189) and a method including the addition of phosphite ion or hypophosphite ion (Japaneae Unexamined Patent Application, First Publication No. 7-213296). However, the cumulative concentration of α-hydroxy acid formed is still not high even if these additive are used. The highest cumulative concentrations are a cumulative concentration of 16.6% by weight of mandelic acid using Alcaligenes sp. strain BC35-2 (Japanese Unexamined Patent Application, First Publication No. 5-192189) and a cumulative concentration of 12% by weight of mandelic acid using Gordona terrae strain MA-1 (Japanese Unexamined Patent Application, First Publication No. 7-213296) indicated in the examples of these respective patent publications.

### DISCLOSURE OF THE INVENTION

It is commonly known among persons having ordinary skills in the art that, in the case of low cumulative concentration of a product or low production rate per microbial catalyst, or in the case a production rate cannot be maintained for a long period of time even if it is somewhat fast, equipment for producing that product are typically complex and large in scale. Consequently, the industrial production of α-hydroxy acid by known methods like those described above has had problems in terms of production efficiency. The object of the present invention is to provide a method for producing α-hydroxy acid ammonium salt using a specific microbial strain that is capable of accumulating α-hydroxy acid ammonium salt to a high concentration while also being able to maintain an industrially satisfactory production rate for a long period of time.

As a result of conducting an extensive search for industrially advantageous microbes that are resistant to inhibition of activity by high concentrations of α-hydroxynitrile or α-hydroxy acid ammonium salt, have durability by which activity is sustained for a long period of time, have the ability to rapidly accumulate highly concentrated α-hydroxy acid ammonium salt, and are among microbes that form α-hydroxy acid ammonium salt from α-hydroxynitrile, the inventors of the present invention found that Arthrobacter sp. NSSC204 (FERM BP-7662), which was newly isolated following mutagenic treatment of Arthrobacter sp. NSSC104 (FERM BP-5829), which was known to previously have the highest level of production ability of α-hydroxy acid ammonium salt, has the aforementioned target activity, thereby leading to completion of the present invention.

Namely, the present invention relates to a method for producing α-hydroxy acid ammonium salt represented by general formula (II) characterized in that, when converting an α-hydroxynitrile represented by RCH(OH)CN of general formula (I) (wherein, R represents a hydrogen atom, a C₁ to C₆ alkyl group which may be substituted, a C₂ to C₆ alkenyl group which may be substituted, a C₁ to C₆ alkoxyl group which may be substituted, an aryl group which may be substituted, an aryloxy group which may substituted or a heterocyclic group which may be substituted) to an α-hydroxy acid ammonium salt represented by RCH (OH) COO⁻NH₄⁺ of general formula (II) (wherein R is the same as previously defined in the general formula (I)), a microbial catalyst originating in a microbial strain is used that is capable of maintaining the average production rate of the α-hydroxy acid ammonium salt represented by general formula (II) at at least 100 µmol/min per g of dry microbial cell weight for 14 days or more without adding fresh microbial catalyst (claim 1), a method for producing α-hydroxy acid ammonium salt represented by general formula (II) according to claim 1, wherein the microbial catalyst originating in a microbial strain is able to accumulate 20 to 60% by weight of the α-hydroxy acid ammonium salt represented by general formula (II) in the reaction system (claim 2), a method for producing α-hydroxy acid ammonium salt represented by general formula (II) according to claim 1 or claim 2, wherein the microbial catalyst originating in a microbial strain is a microbial catalyst originating in a microbial strain belonging to the genus Arthrobacter (claim 3), a method for producing α-hydroxy acid ammonium salt represented by general formula (II) according to claim 3, wherein the microbial strain belonging to the genus Arthrobacter is Arthrobacter sp. NSSC204 or a microbial strain derived from the Arthrobacter sp. NSSC204 (claim 4), a method for producing α-hydroxy acid ammonium salt represented by general formula (II) according to any of claims 1 through 4, wherein the microbial catalyst originating in a microbial strain is a microbial cell, treated microbial cells of said microbe or an extract extracted from said microbe or enzyme isolated from said microbe (claim 5), a method for producing α-hydroxy acid ammonium salt represented by general formula (II) according to any of claims 1 through 5, wherein the C₁ to C₆ alkyl group which may be substituted is a C₁ to C₆ alkylthioalkyl group or a C₁ to C₆ hydroxyalkyl group (claim 6), a method for producing α-hydroxy acid ammonium salt represented by general formula (II) according to any of claims 1 through 5, wherein the aryl group which may be substituted is a phenyl group (claim 7), and a method for producing α-hydroxy acid ammonium salt represented by general formula (II) according to any of claims 1 through 7, wherein the α-hydroxynitrile is lactonitrile, acetone cyanohydrin mandelonitrile or 2-hydroxy-4-methylthiobutyronitrile (claim 8).

In addition, the present invention relates to a microbial strain characterized in that, when converting an α-hydroxynitrile represented by RCH(OH)CN of general formula (I) (wherein, R represents a hydrogen atom, a C₁ to C₆ alkyl group which may be substituted, a C₂ to C₆ alkenyl group which may be substituted, a C₁ to C₆ alkoxyl group which may be substituted, an aryl group which may be substituted, an aryloxy group which may be substituted or a heterocyclic group which may be substituted) to an α-hydroxy acid ammonium salt represented by RCH(OH)COO⁻NH₄⁺ of general formula (II) (wherein R is the same as previously defined in the general formula (I)), is capable of maintaining the average production rate of the α-hydroxy acid ammonium salt represented by general formula (II) at at least 100 µmol/min per g of dry microbial cell weight for 14 days or more without adding fresh microbial catalyst (claim 9), a microbial strain according to claim 9, wherein the microbial strain is able to accumulate 20 to 60% by weight of the α-hydroxy acid ammonium salt represented by general formula (II) in the reaction system (claim 10), a microbial strain according to claim 9 or claim 10, wherein the microbial strain is a microbial strain belonging to the genus Arthrobacter (claim 11), a microbial strain according to claim 11, wherein the microbial strain belonging to the genus Arthrobacter is Arthrobacter sp. NSSC204 (PERM BP-7662) (claim 12), and a microbial strain according to claim 11, wherein the microbial strain belonging to the genus Arthrobacter is a microbial strain derived from Arthrobacter sp. NSSC204 (claim 13).

There are no particular limitations on the method for producing α-hydroxy acid ammonium salt represented by RCH(OH)COO⁻NH₄⁺ of general formula (II) (wherein, R represents a hydrogen atom, a C₁ to C₆ alkyl group which may be substituted, a C₂ to C₆ alkenyl group which may be substituted, a C₁ to C₆ alkoxyl group which may be substituted, an aryl group which may be substituted, an aryloxy group which may be substituted or a heterocyclic group which may be substituted), when it is a method for producing α-hydroxy acid ammonium salt represented by general formula (II), wherein during conversion of an α-hydroxynitrile represented by RCH(OH)CN of general formula (I) (wherein, R is the same as previously defined) to an α-hydroxy acid ammonium salt represented by general formula (II), a microbial catalyst originating in a microbial strain is used that is capable of maintaining the average production rate of α-hydroxy acid ammonium salt represented by general formula (II) at at least 100 µmol/min per g of dry microbial cell weight for 14 days or more without adding fresh microbial catalyst. The microbial catalyst preferably has an average production rate as described above of 200 µmol/min or more, and more preferably an average production rate as described above of 300 µmol/min or more.

In addition, the aforementioned microbial catalyst originating in a microbial strain is preferably a microbial catalyst originating in a microbial strain that is capable of accumulating 20 to 60% by weight of an α-hydroxy acid ammonium salt represented by general formula (II) in the reaction system. An example of a microbial strain capable of accumulating an α-hydroxy acid ammonium salt represented by general formula (II) at 20 to 60% by weight in the reaction system, and capable of maintaining the average production rate of an α-hydroxy acid ammonium salt represented by general formula (II) at 100 µmol/min per g of dry microbial cell weight for 14 days or more without adding fresh microbial catalyst is a microbial strain belonging to the genus Arthrobacter. Specific examples of this microbial strain include Arthrobacter sp. NSSC204 or a microbial strain derived from said strain.

The aforementioned Arthrobacter sp. NSCC204 was newly isolated as a result of performing mutagenic treatment on Arthrobacter sp. NSSC104 (FERM BP-5829), which was previously known to have the highest level of production ability of α-hydroxy acid ammonium salt. The new strain of Arthrobacter sp. NSSC104 was discovered by the inventors of the present invention.

Arthrobacter sp. NSSC104 (FERM BP-5829) was deposited on February 6, 1996 at the Independent Administrative Corporation, National Institute of Advanced Industrial Science and Technology (Chuo No. 6, 1-1-1 Higashi, Tsukuba City, Ibaraki Prefecture, Japan), and its microbial properties are described in WO 97/32030.

Arthrobacter sp. NSSC204 (FERM BP-7662) was similarly deposited on June 22, 2000 at the Independent Administrative Corporation, National Institute of Advanced Industrial Science and Technology (Chuo No. 6, 1-1-1 Higashi, Tsukuba City, Ibaraki Prefecture, Japan), and its microbial properties are as described below.

| | |
|---|---|
| Morphotype | Polymorphic rods |
| Gram staining | Positive |
| Rod-coccus cycle | Positive |
| Spore formation | Negative |
| Mobility | Negative |
| Cell wall diamino acid | Lysine |
| Oxygen behavior | Aerobic |
| Catalase | + |
| DNA degradation | + |
| Gelatin liquefaction | + |
| Starch degradation | + |
| Casein degradation | + |
| Auxotrophy | Negative |
| Glycolyl test | - |
| Quinone system | MK-9 (H2) |

As a result of searching for the aforementioned micrological properties based on Bergey's Manual of Systematic Bacteriology (1986), strain NSSC204 was identified as a new strain belonging to the genus Arthrobacter. In addition, this strain NSSC204 exhibits concentration tolerance to α-hydroxynitrile (I) and/or α-hydroxy acid ammonium salt (II) even at concentrations of 50 to 60% by weight.

Although there are no particular limitations on the microbial strain as claimed in the present invention provided it is a microbial strain which, during conversion of α-hydroxynitrile represented by general formula (I) to α-hydroxy acid ammonium salt represented by general formula (II), is capable of maintaining the average production rate of α-hydroxy acid ammonium salt represented by general formula (II) at at least 100 µmol/min per g of dry microbial cell weight for 14 days or more without adding fresh microbial catalyst, a microbial strain having activity of 200 µmol/min or more, and particularly 300 µmol/min or more, for the aforementioned average production rate is preferable for the aforementioned microbial strain. Furthermore, a microbial strain capable of accumulating α-hydroxy acid ammonium salt represented by general formula (II) at 20 to 60% by weight in the reaction system is more preferable.

There are no particular limitations on the method for establishing the microbial strain of the present invention, and although examples of methods that can be used include screening from the soil and the like, mutagenic methods using radiation, ultraviolet rays or mutagens, gene recombination methods and cell fusion methods, mutagenic treatment is preferable in terms of the simplicity of the procedure. As an example of a mutagenic method, after performing mutagenic treatment according to an ordinary method using radiation, ultraviolet rays or infrared rays on a known microbial strain having the ability to convert α-hydroxynitrile to α-hydroxy acid ammonium salt, a strain that is superior in terms of the amount of production of α-hydroxy acid ammonium salt produced from α-hydroxynitrile (superior cumulative concentration and production rate) or a microbial strain exhibiting tolerance in a highly concentrated solutions of α-hydroxynitrile and α-hydroxy acid ammonium salt can be obtained by screening. In addition, a desired microbial strain can be obtained by performing secondary screening under more rigid conditions on microbial strains selected by primary screening, or by performing secondary screening under more rigid conditions after having repeated mutagenic treatment on microbial strains selected by primary screening.

Culturing of microbes as claimed in the present invention is carried out in normal medium containing an enzyme inducing substance, and carbon source, nitrogen source, inorganic ions and, as necessary, organic nutrient source capable of being assimilated by the microbes. Examples of enzyme inducing substances include nitrile compounds such as isobutyronitrile, and cyclic amide compounds such as ε-caprolactam. Examples of carbon sources that can be suitably used include carbohydrates such as glucose, alcohols such as ethanol and organic acids. Examples of nitrogen sources include amino acids, nitrates and ammonium salts. Examples of inorganic ions which can be used in the present invention as necessary include phosphate ions, potassium ions, magnesium ions, sulfate ions and iron ions. Examples of organic nutrients that can be suitably used include vitamins, amino acids as well as corn steep liquor, yeast extract, polypeptones and beef extract in which they are contained. Culturing should be carried out under aerobic conditions while controlling to a pH of 6 to 9 and suitable temperature range of 25-37°C.

Examples of microbial catalysts originating in microbial strains that are used in the hydrolysis reaction from α-hydroxynitrile to α-hydroxy acid ammonium salt of a microbe of the present invention include microbial cells, treated microbial cells of said microbe or an extract from the microbe or enzyme isolated from said microbe. The microbial catalyst can be prepared by collecting microbial cells cultured in the manner described above, if necessary, in the form of microbial cell treatment products such as immobilized cells, crude enzyme, immobilized crude enzyme. In the case of immobilizing microbial cells or enzyme, ordinary immobilization techniques can be applied such as the carrier binding method and entrapment methods. In the case of preparing a crude enzyme, after crushing the microbial cells with ultrasonic waves, a high-pressure homogenizer or the like, ordinary enzyme purification techniques can be applied such as salting out with ammonium sulfate and chromatography.

Hydrolysis from α-hydroxynitrile to α-hydroxy acid ammonium salt is carried out by allowing the microbial catalyst to contact the α-hydroxynitrile in an aqueous solvent. Microbial cells are normally used at a concentration of 0.01 to 10% by weight in terms of their dry weight, and following completion of the reaction, are recovered by filtration, centrifugal separation or ultrafiltration membrane concentration so that they can be used repeatedly for the hydrolysis reaction. Although examples of the aforementioned aqueous solvent include water and aqueous solutions containing buffers or other salts or organic solvents, these may be separated into two phases.

Preferable examples of C₁ to C₆ alkyl groups which may be substituted in the α-hydroxynitrile represented by general formula (I) used in the present invention (wherein, R represents a hydrogen atom, a C₁ to C₆ alkyl group which may be substituted, a C₂ to C₆ alkenyl group which may be substituted, a C₁ to C₆ alkoxyl group which may be substituted, an aryl group which may be substituted, an aryloxy group which may be substituted or a heterocyclic group which may be substituted) include a C1 to C6 alkylthioalkyl group or C1 to C6 hydroxyalkyl group, while preferable examples of aryl groups which may be substituted include a phenyl group, and specific examples of the α-hydroxynitrile represented by general formula (I) include lactonitrile, acetone cyanohydrin, mandelonitrile and 2-hydroxy-4-methylthiobutyronitrile. These α-hydroxynitriles (I) are normally used in the reaction at a concentration of 0.1 to 50% by weight, and may be added during the reaction necessary. The reaction pH may be maintained between 5 and 10 with a suitable buffer or acid and base. The reaction temperature may normally be maintained at 4 to 50°C and preferably at 20 to 40°C. The reaction temperature may be changed as necessary, and a reaction system may also be used in which the temperature is raised or lowered gradually.

There are no particular limitations on the reaction time of the hydrolysis reaction from α-hydroxynitrile to α-hydroxy acid ammonium salt. Normally, by a reaction time of 6 to 120 hours, the α-hydroxy acid ammonium salt (II) corresponding to the α-hydroxynitrile (I) used in the reaction solution, examples of the salt include ammonium lactate, ammonium 2-hydroxyisobutyrate, ammonium mandelate and ammonium 2-hydroxy-4-methylthiobutyrate, accumulates to a high concentration of, for example, 20% by weight or more. The microbe used in the reaction is able to be used repeatedly in the hydrolysis reaction without any substantial decrease in activity. The product of the reaction can be separated and purified in accordance with general methods such as concentration and extraction, and the ammonium can be separated as necessary by a method such as organic solvent extraction under acidic conditions, thermal decomposition and the like.

### BEST MEANS FOR CARRYING OUT THE INVENTION

The following provides a more detailed explanation of the present invention through its examples. The present invention is not limited in any way by these examples.

### Example 1

2 ml of medium containing 0.3% beef broth, 0.5% peptone and 0.5% salt were placed in a test tube, and 20 ml of medium having the composition shown below were placed in a 100 ml volumetric Erlenmeyer flask equipped with a baffle, and each was sterilized for 15 minutes at 121°C. One loopful of Arthrobacter sp. NSSC204 was inoculated into the test tube including 2 ml of the medium, and after shake culturing overnight at 30°C, 0.2 ml thereof were subcultured to the Erlenmeyer flask equipped with baffle followed by additionally shake culturing at 30°C for 5 days. The microbial cells obtained by centrifuging this culture liquid were washed with physiological salt solution, and 4% by weight of the microbial cells in terms of their dry weight were suspended in 0.1 M phosphate buffer (pH 7.5). Next, 2-hydroxy-4-methylthiobutyronitrile was added to a final concentration of 237 mM, and the hydrolysis reaction was carried out while shaking gently at 30°C. The same amount of 2-hydroxy-4-methylthiobutyronitrile was repeatedly added seven times at one hour intervals after first addition of 2-hydroxy-4-methylthiobutyronitrile followed by adding 2-hydroxy-4-methylthiobutyronitrile eight times at 1.5 hour intervals so that the reaction was carried out for a total of 19 hours.

| | |
|---|---|
| Corn steep liquor | 1.0% (sterilized separately) |
| Sucrose | 1.0% (sterilized separately) |
| Potassium dihydrogenphosphate | 0.1% |
| Dipotassium hydrogenphosphate | 0.1% |
| Sodium chloride | 0.02% |
| Magnesium sulfate heptahydrate | 0.02% |
| Ferrous sulfate | 0.001% (sterilized separately) |
| ε-caprolactam | 0.5% |
| pH | 7.2 (adjusted with 2 N sodium hydroxide) |

Following completion of the reaction, as a result of removing the microbial cells by centrifuging the reaction solution and quantifying the concentration of 2-hydroxy-4-methylthiobutyric acid contained in the centrifuged supernatant using high-performance liquid chromatography (column: TSKgel ODS-80TM, carrier: acetonitrile/water/trifluoroacetic acid = 80/20/0.1), 58.9% by weight of ammonium 2-hydroxy-4-methylthiobutyrate were confirmed to have accumulated (yield: 98%). When the reaction was carried out under identical conditions using Arthrobacter sp. strain NSSC104, the amount of a single addition of 2-hydroxy-4-methylthiobutyronitrile was 200 mM as the initial final concentration, and the cumulative concentration of ammonium 2-hydroxy-4-methylthiobutyrate was 48.8% by weight corresponding to a yield of 96%.

### Example 2

A reaction was carried out to produce ammonium 2-hydroxy-4-methylthiobutyrate at a constant rate by controlling the rise in temperature using microbes obtained by feed culturing. Pre-culturing medium in the form of 200 ml of liquid medium containing 0.5% by weight of yeast extract, 0.5% by weight of glucose, 0.5% by weight of ε-caprolactam, 0.1% by weight of K₂HPO₄, 0.1% by weight of KH₂PO₄, 0.02% by weight of magnesium sulfate heptahydrate, 0.1% by weight of magnesium chloride and 0.001% by weight of ferrous sulfate heptahydrate adjusted to pH 7.2 with 1 N sodium hydroxide were placed in a 3 liter volumetric Erlenmeyer flask followed by sterilizing for 20 minutes at 120°C (only the ferrous sulfate was sterilized separately by filtration and then added to the medium). This pre-culturing medium was then inoculated with Arthrobacter sp. strain NSSC204 followed by pre-culturing by shake culturing for 3 days at 33°C.

Next, an aqueous solution was prepared that contained 20% by weight of corn steep liquor and was adjusted to pH 7.0 with 10N sodium hydroxide, and the supernatant from which the insoluble matter that formed was removed by centrifugation was used as corn steep liquor extract. 2.9 liters of liquid medium composed of 20.5% by weight of corn steep liquor extract, 0.5% by weight of glucose and 1% by weight of ε-caprolactam were prepared in a 10 liter volumetric jar fermenter. Sterilization was performed by filtration sterilization for the corn steep liquor extract and by heat sterilization for 20 minutes at 120°C for the other medium components. This growth medium was inoculated with the culture liquid obtained in the aforementioned pre-culturing and aeration stir cultured at 33°C. 2.5 liters of liquid medium comprising of 95.5% by weight of the corn steep liquor extract prepared by the same sterilization method as described above and 2.2% by weight of glucose were fed between 6 and 12 hours after the start of culturing. The dissolved oxygen concentration recovered and the logarithmic growth phase ended at about 1 hour after completion of feeding. Immediately after completion of the logarithmic growth phase, 560 ml of 50% by weight glucose solution preliminarily sterilized for 20 minutes at 110°C were fed over the course of about 10 hours. Following addition of glucose, aging culturing was continued for 3 days under aerated stirring conditions at 33°C. The microbes were recovered from this culture liquid by centrifugation and then washed with physiological saline to obtain 660 g of wet microbial cells (132 g as the dry microbial cell weight).

86.0 g (17.2 g as the dry microbial cell weight) of wet microbial cells of the microbial catalyst Arthrobacter sp. NSSC204 obtained by the aforementioned culturing method were added to a 300 ml volumetric stirring reaction tank equipped with an ultrafiltration (UF) membrane separator, high-performance liquid chromatograph provided with an automatic diluter and auto-injector and pH controller, and then diluted with 189.2 ml of water.
2-hydroxy-4-methylthiobutyronitrile was then added continuously to this reaction tank at the rate of 0.409 g/min and at a reaction temperature of 21°C, and at the point a total of 51. 8 g had finished being added, continuous addition of water at the rate of 1.701 g/min was begun. Simultaneous to separating and recovering the microbial catalyst with the membrane separator and returning it to the reaction tank while continuing continuous addition of 2-hydroxy-4-methylthiobutyronitrile at the rate of 0.409 g/min, the permeated filtrate was discharged at the rate of 2.111 g/min. In addition, 28% aqueous ammonia was added throughout the reaction to maintain the pH at 7.0.

During this continuous reaction, the concentration of 2-hydroxy-4-methylthiobutyronitrile in the reaction solution was detected by online high-performance liquid chromatography, and the reaction temperature was raised gradually so that the concentration reached about 0.2% by weight. In this manner, a membrane-permeated filtrate containing 22.9% by weight (average value) of ammonium 2-hydroxy-4-methylthiobutyrate was discharged at the aforementioned constant rate. When this reaction was continued for 27 days, the reaction temperature rose to 35°C. The total amount of membrane-permeated filtrate was 85.1 kg. Moreover, following completion of this continuous reaction for 27 days, the residual liquid present in the reaction tank and reaction lines was membrane-concentrated and washed to separately obtain 534.5 g of washed membrane-permeated filtrate. When the resulting membrane-permeated filtrate and washed membrane-permeable filtrate were analyzed by high-performance liquid chromatography, the 2-hydroxy-4-methylthiobutyric acid product was present at 20.0% by weight and 9.35% by weight, respectively, and since the purity of the 2-hydroxy-4-methylthiobutyronitrile used as the substrate was 95.0%, the molar yield of the product was calculated to be 95.0%. Based on the aforementioned reaction results, calculation of the average production rate per microbial catalyst of the ammonium 2-hydroxy-4-methylthiobutyrate yielded a value of 174.5 µmol/min/g of dry microbial cell weight (21°C→35°C, 27 days).

### Comparative Example 1

When a reaction was carried out under the same conditions as Example 2 using for the microbial catalyst Arthrobacter sp. strain NSSC104 obtained by culturing under the same conditions as Example 2, the following results were obtained.
Amount of microbial catalyst used: 86.0 g (17.2 g as dry microbial cell weight, same as Example 2)
Reaction temperature: 21°C→35°C (27 days, same as Example 2)
Addition rate of 2-hydroxy-4-methylthiobutyronitrile added: 0.227 g/min
Water addition rate: 0.945 g/min
Filtrate discharge rate: 1.173 g/min
Average cumulative concentration of ammonium 2-hydroxy-4-methylthiobutyrate: 22.8% by weight
Amount of filtrate discharged: 47.3 kg/27 days
Average production rate per microbial catalyst of ammonium 2-hydroxy-4-methylthiobutyrate: 96.6 µmol/min/g of dry microbial cell weight

### Example 3

A reaction that continuously produces ammonium 2-hydroxy-4-methylthiobutyrate at a constant temperature was carried out using microbes obtained by feed culturing.

86.0 g (17.2 g as the dry microbial cell weight) of wet microbial cells of the microbial catalyst Arthrobacter sp. NSSC204 obtained by the aforementioned culturing method were added to a 300 ml volumetric stirring reaction tank equipped with an ultrafiltration (UF) membrane separator, high-performance liquid chromatograph provided with an automatic diluter and auto-injector and pH controller, and then diluted with 189.2 ml of water.
2-hydroxy-4-methylthiobutyronitrile was then added continuously to this reaction tank at the rate of 0.974 g/min and at a reaction temperature of 35°C, and at the point a total of 51. 8 g had finished being added, continuous addition of water at the rate of 4.052 g/min was begun. Simultaneous to separating and recovering the microbial catalyst with the membrane separator and returning it to the reaction tank while continuing continuous addition of 2-hydroxy-4-methylthiobutyronitrile at the rate of 0.974 g/min, the permeated filtrate was discharged at the rate of 5.028 g/min. 28% aqueous ammonia was added throughout the reaction to maintain the pH at 7.0.

During this continuous reaction, the concentration of 2-hydroxy-4-methylthiobutyronitrile in the reaction solution was detected by online high-performance liquid chromatography, and the addition rates of 2-hydroxy-4-methylthiobutyronitrile and water were gradually decreased so that the concentration reached about 0.2% by weight. The discharge rate of the membrane-permeated filtrate was also lowered accompanying these decreases. After continuing this reaction for 18 hours under these conditions, the addition rate of 2-hydroxy-4-methylthiobutyronitrile was 0.902 g/min and the discharge rate of the membrane-permeated filtrate was 4.658 g/min at completion of the reaction. The total amount of 2-hydroxy-4-methylthiobutyronitrile added after the start of the continuous reaction was 1.01 kg, and the total amount of membrane-permeated filtrate was 5.23 kg. When the resulting membrane-permeated filtrate was analyzed by high-performance liquid chromatography, the 2-hydroxy-4-methylthiobutyric acid product was present at 20.7% by weight and since the purity of the 2-hydroxy-4- methylthiobutyronitrile used as the substrate was 95.0%, the molar yield of the product was calculated to be 98.5%. Based on the aforementioned reaction results, calculation of the average production rate per microbial catalyst of the ammonium 2-hydroxy-4-methylthiobutyrate yielded a value of 388.5 µmol/min/g of dry microbial cell weight (35°C, 18 hours).

### INDUSTRIAL APPLICABILITY

According to the present invention, the use of a microbe having concentration tolerance to α-hydroxynitrile (I) and/or α-hydroxy acid ammonium salt (II) as well as durability wherein activity is sustained for a long period of time allows α-hydroxy acid ammonium salt (II) to be accumulated at high concentrations while also enabling a microbial catalyst to be used repeatedly, thereby making it possible to efficiently produce α-hydroxy acid ammonium salt (II).

## Claims

1. A method for producing α-hydroxy acid ammonium salt represented by RCH(OH)COO⁻NH₄⁺ of general formula (II) **characterized in that**, when converting an α-hydroxynitrile represented by RCH(OH)CN of general formula (I) (wherein, R represents a hydrogen atom, a C₁ to C₆ alkyl group which may be substituted, a C₂ to C₆ alkenyl group which may be substituted, a C₁ to C₆ alkoxyl group which may be substituted, an aryl group which may be substituted, an aryloxy group which may be substituted or a heterocyclic group which may be substituted) to an α-hydroxy acid ammonium salt represented by RCH(OH)COO⁻NH₄⁺ of general formula (II) (wherein R is the same as previously defined), a microbial catalyst originating in a microbial strain is used that is capable of maintaining the average production rate of the α-hydroxy acid ammonium salt represented by general formula (II) at at least 100 µmol/min per g of dry microbial cell weight for 14 days or more without adding fresh microbial catalyst.

2. A method for producing α-hydroxy acid ammonium salt represented by general formula (II) according to claim 1, wherein the microbial catalyst originating in a microbial strain is able to accumulate 20 to 60% by weight of the α-hydroxy acid ammonium salt represented by general formula (II) in the reaction system.

3. A method for producing α-hydroxy acid ammonium salt represented by general formula (II) according to claim 1 or claim 2, wherein the microbial catalyst originating in a microbial strain is a microbial catalyst originating in a microbial strain belonging to the genus Arthrobacter.

4. A method for producing α-hydroxy acid ammonium salt represented by general formula (II) according to claim 3 wherein, the microbial strain belonging to the genus Arthrobacter is Arthrobacter sp. NSSC204 or a microbial strain derived from the strain.

5. A method for producing α-hydroxy acid ammonium salt represented by general formula (II) according to any of claims 1 through 4, wherein the microbial catalyst originating in a microbial strain is a microbial cell, treated microbial cells of the microbe, an extract extracted from the microbe or enzyme isolated from the microbe.

6. A method for producing α-hydroxy acid ammonium salt represented by general formula (II) according to any of claims 1 through 5, wherein the C₁ to C₆ alkyl group which may be substituted is a C₁ to C₆ alkylthioalkyl group or a C₁ to C₆ hydroxyalkyl group.

7. A method for producing α-hydroxy acid ammonium salt represented by general formula (II) according to any of claims 1 through 5 wherein, the aryl group which may be substituted is a phenyl group.

8. A method for producing α-hydroxy acid ammonium salt represented by general formula (II), wherein the α-hydroxynitrile is lactonitrile, acetone cyanohydrin, mandelonitrile or 2-hydroxy-4-methylthiobutyronitrile.

9. A microbial strain **characterized in that**, when converting an α-hydroxynitrile represented by RCH(OH)CN of general formula (I) (wherein, R represents a hydrogen atom, a C₁ to C₆ alkyl group which may be substituted, a C₂ to C₆ alkenyl group which may be substituted, a C₁ to C₆ alkoxyl group which may be substituted, an aryl group which may be substituted, an aryloxy group which may be substituted or a heterocyclic group which may be substituted) to an α-hydroxy acid ammonium salt represented by RCH(OH)COO⁻NH₄⁺ of general formula (II) (wherein R is the same as previously defined), is capable of maintaining the average production rate of the α-hydroxy acid ammonium salt represented by general formula (II) at at least 100 µmol/min per g of dry microbial cell weight for 14 days or more without adding fresh microbial catalyst.

10. A microbial strain according to claim 9 wherein, the microbial strain is able to accumulate 20 to 60% by weight of the α-hydroxy acid ammonium salt represented by general formula (II) in the reaction system.

11. A microbial strain according to claim 9 or claim 10, wherein the microbial strain is a microbial strain belonging to the genus Arthrobacter.

12. A microbial strain according to claim 11, wherein the microbial strain belonging to the genus Arthrobacter is Arthrobacter sp. NSSC204 (FERM BP-7662).

13. A microbial strain according to claim 11, wherein the microbial strain belonging to the genus Arthrobacter is a microbial strain derived from Arthrobacter sp. NSSC204.
